# EUROPEAN PATENT APPLICATION

(11) **EP 1 084 724 A1**
(43) Date of publication of application: **21.03.2001**
(21) Application number: 00307910.0
(22) Date of filing: 13.09.2000
(51) Int. Cl.: A61M 5/31, A61M 5/315, A61B 10/00

(54) **Syringe**

(30) Priority: 15.09.1999 GB 9921764; 07.07.2000 GB 0016585
(71) Applicant: Kay, Matthew Red, Bumley BB10 4HU (GB)
(72) Inventor: Kay, Matthew Red, Bumley BB10 4HU (GB)
(74) Representative: Brandon, Paul Laurence

(57) **Abstract**

The present invention provides a syringe (2) designed to utilise a vacuum to aid withdrawal of a liquid from a body and thereby simplify the process of liquid withdrawal.

## Description

### Field of the Invention

The present invention relates to syringes, in particular, to syringes comprising a barrel and plunger which can be fitted with a hollow needle.

### Background of the Invention

Syringes are frequently used to withdraw samples of fluids from a body. Syringes are also used to drain a reservoir of liquid from a region of a body.

Conventional syringes used for these purposes generally comprise a hollow barrel fitted with a plunger and hollow needle. In use the plunger is pushed into the barrel so that the plunger is in a fully inserted condition, the needle is then inserted into the body. The needle of the syringe must be guided into the body until the tip of the needle is inserted into a liquid reservoir to be sampled/drained.

In use, it is often important to prevent the needle tip being inserted too far into the body. Therefore, once the needle tip has been inserted into the body the plunger is withdrawn slightly into the barrel of the syringe. The plunger can only be withdrawn a small amount, because the needle tip is surrounded by solid or substantially solid material. Withdrawal of the plunger into the barrel creates a partial vacuum between the needle tip and the plunger head. Therefore, when the needle tip is inserted into a liquid reservoir in the body a quantity of liquid is automatically sucked into the barrel of the syringe by means of the vacuum created between the needle tip and the plunger head. The user then knows when the needle tip is inserted into the liquid reservoir and thereby knows when to cease insertion of the needle into the body.

In order to produce this effect the plunger must be maintained in the slightly withdrawn condition throughout insertion and guidance of the needle through the body. To maintain the plunger in this slightly withdrawn condition the user must maintain a continuous pulling force on the plunger. Use in such a manner requires both hands, one to maintain the piston in the correct location and the other to support and guide the barrel.

It is difficult to guide the syringe whilst one hand is being used to maintain the plunger in a slightly withdrawn condition. Such syringes are disadvantageous in that their proper use depends on the degree of skill of the person carrying out the process.

European Patent Application No. 94202117 discloses a self-aspirating syringe comprising a plunger having cooperating male and female components which are covered by an elastomeric diaphragm sheath. In use, the plunger is inserted fully into the barrel of the syringe to expel the air from the barrel. The force applied to the plunger to insert it into the barrel is sufficient to deform the elastomeric sheath when the plunger head reaches the end of the barrel. Once the needle tip has been located inside a body, the insertion force is released from the plunger flange causing the elastomeric sheath to retract to its original shape and thereby generate a small negative pressure between the needle and the plunger head. When the tip of the needle is inserted into a liquid reservoir a small amount of liquid will be drawn into the barrel of the syringe.

The syringe disclosed in European Patent Application No. 94202117 advantageously does not require one hand to maintain the plunger in slightly retracted position, thus leaving both hands free to guide the needle into the body. However, the plunger design is complicated and costly to manufacture. In view of the enormous number of conventional syringes used for this purpose in the United Kingdom alone each year, it is important that the syringe design is simple and cheap to manufacture.

It is an object of preferred embodiments of the present invention to provide an improved syringe.

### Summary of the Invention

The present invention provides a syringe comprising a hollow barrel, providing a chamber, having a needle attachment means and a plunger which is moveable within said chamber, said plunger comprising a plunger head and a rod, said plunger head being locatable inside said chamber, said plunger head being adapted to form a fluid tight fit with a wall of said chamber, and a closure means being arranged such that when said plunger is located within said chamber, said closure means is situated to the opposite side of said plunger head to which said needle attachment means is located, said closure means forming a fluid tight fit between said chamber and said plunger rod, wherein said needle attachment means is fixedly located relative to said hollow barrel.

Suitably, the closure means comprises a collar. Suitably, the collar projects between the hollow barrel of the syringe and the rod of the plunger. Preferably, the collar is located within the chamber. Alternatively, the collar may be located at the end of the hollow barrel.

The collar may project from the chamber wall. The collar may be formed by an extension of the chamber wall. Alternatively, the collar may be separate from the hollow barrel, but may be either permanently or temporarily fixed to the inner surface of the hollow barrel, which inner surface provides the chamber wall.

Alternatively, the collar may project from the end of the hollow barrel. The collar may be either permanently or temporarily attached to the end of the hollow barrel. Alternatively, the collar may be provided by an extension of the hollow barrel.

The closure means may comprise any suitable material. Preferably, the closure means comprises an elastomeric material.

Suitably, the plunger further comprises a flange attached to the end of the rod that is distal the plunger head.

Preferably, the hollow barrel of the syringe has an end wall at which said needle attachment means is located.

Suitably, said needle attachment means comprises a hollow projection having an opening in each end. Preferably, the hollow projection and the chamber are in fluid flow communication.

Suitably, the needle attachment means is an integral part of the hollow barrel of the syringe. Suitably, the needle attachment means is provided by an extension of the hollow barrel of the syringe. Preferably, the needle attachment means is provided by a moulded extension of the hollow barrel of the syringe.

Suitably, said syringe is designed such that a needle can be immovably attached relative to the hollow body of the syringe.

Suitably, when a needle is attached to the needle attachment means, the needle cannot be withdrawn inside the hollow barrel by means of the plunger.

Suitably, a needle attached to the needle attachment means is not withdrawn into the chamber of the syringe by means of a vacuum generated inside the chamber.

A syringe in accordance with the present invention may further comprise a fluid outlet. Suitably, the fluid outlet is located between the needle attachment means and the other end of the hollow body. Preferably, the fluid outlet is located on the end wall of the hollow barrel. Suitably said fluid outlet is located adjacent the needle attachment means. However, the fluid outlet may be located in a side wall of the hollow barrel. Preferably, the fluid outlet is located in the region of the end wall of the hollow barrel.

If the syringe comprises fluid outlet the needle attachment means and/or the fluid outlet may further comprise valve means. The valve means should be adapted to restrict fluid flow through the needle attachment means and the fluid outlet to one direction only.

In this case, fluid flow through the needle attachment means is preferably from the exterior of the needle into the chamber of the syringe. Fluid flow through the fluid outlet is preferably from the chamber of the syringe to the exterior of the hollow barrel of the syringe.

The present invention further provides a method of operating a syringe in accordance with the present invention comprising the following steps:
(a) attaching a needle to said needle attachment means;
(b) locating said plunger head of said plunger inside the chamber;
(c) applying and maintaining an external pressure on said plunger in order to move said plunger head into contact with said end wall of said hollow barrel;
(d) inserting said needle into a body;
(e) releasing the external pressure on said plunger, and
(f) guiding said needle into said body until the tip of said needle contacts a liquid reservoir.
Suitably, when the syringe comprises a fluid outlet, the method further comprises the step of:
(g) reapplying an external pressure to said plunger in order to force liquid collected into said chamber out of said fluid outlet.

In operation of a syringe in accordance with the present invention, a hollow needle is attached to the needle attachment means and the plunger head of the plunger is located inside the chamber of the syringe.

An external pressure is then applied to the plunger to move the plunger head through the full length of the chamber into a fully inserted condition, such that the plunger head contacts the end wall of the hollow barrel. As the plunger is inserted into the chamber a vacuum is generated inside the chamber in the region between the plunger head and the closure means.

Whilst the plunger is maintained in the fully inserted condition, the needle is inserted into a body. The external pressure applied to the plunger may now be released. The vacuum in the chamber causes the plunger head to withdraw slightly from the fully inserted condition. A partial vacuum is then generated between the needle tip and the plunger head. The plunger head is prevented from withdrawing fully because the needle tip is located in a solid or substantially solid material, which cannot be drawn into the needle.

The needle is now guided through the body until the tip of the needle contacts a liquid reservoir. When the needle tip contacts the liquid reservoir, liquid is automatically withdrawn into the chamber and the plunge head withdraws fully by virtue of the vacuum in the chamber.

If the syringe comprises a fluid outlet, when the needle tip contacts the liquid reservoir, the liquid is drawn into the chamber as described above. By inserting the plunger head back into the barrel, the liquid passes out of the chamber through the fluid outlet. A tube can be attached to the fluid outlet to direct the liquid to a suitable receptacle.

Whilst the plunger head may make contact with the end wall of the hollow barrel of the syringe when in the fully inserted condition, the plunger head loses contact with the end wall of the hollow barrel when the plunger head is withdrawn from the fully inserted condition.

If the needle attachment means is located at the end wall of the hollow barrel, the plunger head may contact the needle attachment means when in the fully inserted condition. However, again the plunger head loses contact with the needle attachment means when the plunger head withdraws from the fully inserted condition.

Preferred embodiments of the present invention advantageously provide a syringe that does not require a hand to control the plunger, but instead leaves both hands free to guide the needle attached to the syringe to the desired location within a body.

A syringe having a fluid outlet can advantageously be used to drain a quantity of liquid from a body. This is particularly advantageous is the quantity of liquid required to be drained or the size of the sample required is larger than the available volume of the chamber of the syringe.

Furthermore, a syringe in accordance with the present invention having a fluid outlet and being fitted with valves to control the direction of fluid flow can advantageously be adapted to prevent accidental injection of a gas into a body via a needle attached to the needle attachment means.

### Brief Description of the Drawings

The present invention will now be described, by way of example only, with reference to the following drawings, in which:-
Figure 1 is a cross-sectional view of a first embodiment of a syringe in accordance with the present invention, in a first position.
Figure 2 is a cross-sectional view of the syringe of figure 1 in a second position.
Figure 3 is a cross-sectional view of a second embodiment of a syringe in accordance with the present invention.
Figure 4 is a cross-sectional view of a third embodiment of a syringe in accordance with the present invention.

### Description of the Preferred Embodiments

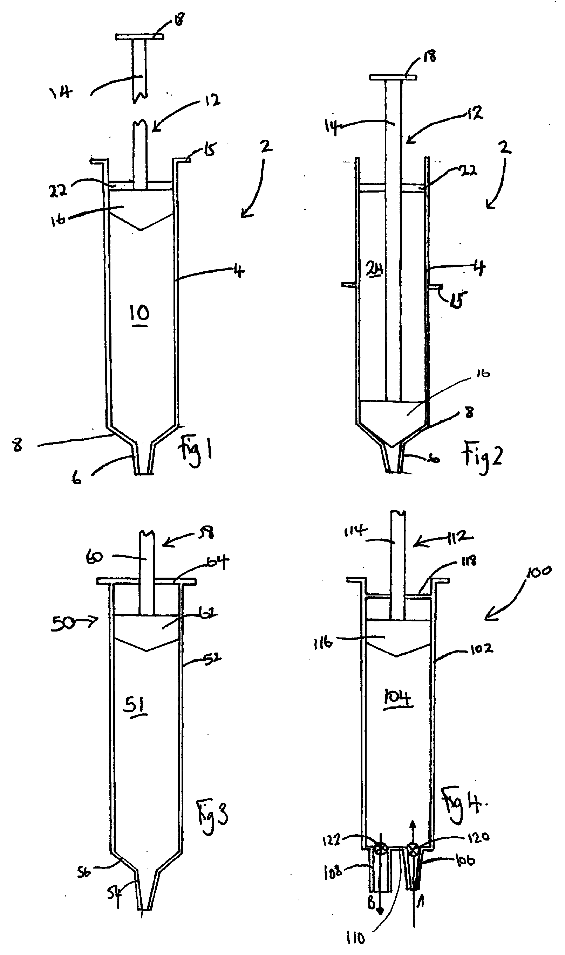

The syringe 2 of figures 1 and 2 comprises a hollow barrel 4 having a needle attachment means 6 attached to an end wall 8, which hollow barrel 4 provides a chamber 10. The needle attachment means 6 is fixedly located relative to the hollow barrel 4. The needle attachment means is provided by an extension of the end wall 8.

The syringe 2 further comprises a plunger, generally designated by reference numeral 12, which plunger 12 comprises a rod 14 a plunger head 16 and a flange 18.

Finally, the syringe 2 further comprises a closure means 22 provided by a collar. The collar 22 is attached to inner wall of the hollow barrel 4. The collar 22 is thereby located inside the chamber 10. The collar 22 forms a fluid tight fit with the rod 14 of the plunger.

Figure 1 illustrates the syringe 2 in a first condition, in which the plunger head 16 is withdrawn towards the end of the chamber 10 which is distal the needle attachment means 6. In this embodiment the withdrawal of the plunger head 16 is limited by the presence of the collar 22 within the chamber.

Figure 2 illustrates the syringe 2 in a second condition, wherein the plunger head 16 is fully inserted into the chamber 10. In this embodiment, insertion of the plunger head 16 is limited by the presence of the end wall 8 of the hollow barrel 4. There is a fluid tight fit between the plunger head 16 and the wall of the chamber 10. There is also a fluid tight fit, provided by the closure means 22, between the chamber 10 and the rod 14.

Figure 2 also illustrates an alternative location for a flange 15. Locating the flange 15 further down the barrel 4 towards the needle attachment means 6, rather than at the end of the barrel 4 as shown in figure 1, may make the syringe 2 easier to use.

In operation of the syringe 2 of figures 1 and 2, insertion of the plunger head 16 into the chamber 10 by application of an external pressure will cause a vacuum to be generated in the section 24 of the chamber between the plunger head 16 and the closure means 22. Insertion of the needle tip into a body (not shown) followed by subsequent removal of the external pressure from the plunger 12, will cause the plunger head 16 to withdraw slightly from the fully inserted condition. A partial vacuum will thus be generated in the region between the needle tip and the plunger head 16. When the needle tip is inserted into a liquid reservoir (not shown), the plunger head 16 will be withdrawn into the chamber 10 by means of the vacuum. Liquid will thus be drawn into the chamber 10.

The needle attachment means 6 and any needle (not shown) attached thereto, will not be withdrawn into the chamber 10 when the plunger head 16 is caused to withdrawn into the chamber 10 by means of the vacuum.

The syringe 50 of figure 3 comprises a hollow barrel 52 defining a chamber 51 and having a needle attachment means 54 and an end wall 56. The syringe 50 also comprises a plunger 58, having a rod 60 and a plunger head 62. The syringe 50 further comprises a closure means 64, providing a fluid tight fit between the chamber 51 and the rod 60.

In this embodiment of the invention the closure means 64 comprises a flange, which flange is an extension of the end of the hollow barrel 52.

The needle attachment means 54 again comprises a hollow protrusion which is an integral part of the hollow barrel 52 of the syringe 50.

The syringe 50 illustrated in figure 3 operates in the same manner as the syringe 2 discussed above in relation to figures 1 and 2.

Figure 4 illustrates a syringe 100 comprising a hollow barrel 102 that defines a chamber 104, which hollow barrel 102 further comprises a needle attachment means 106, a fluid outlet 108 and an end wall 110. The needle attachment means 106 comprises a moulded extension of the hollow barrel 102 and is therefore fixedly located relative to the hollow barrel 102.

The syringe 100 further comprises a plunger 112, comprising a rod 114 and a plunger head 116, and a closure means 118.

The closure means 118 comprises a flange which provides a fluid tight fit between the chamber 104 and the rod 114. The flange 118 extends from the side wall of the hollow barrel 102. The flange 118 is located inside the chamber 104.

The syringe 100 of figure 4 further comprises valve means 120 and 122 associated with the needle attachment means 106 and the fluid outlet respectively. Valve means 120 can be used to restrict fluid flow through the needle attachment means 106 to the direction shown by arrow A. Valve means 122 can be used to restrict fluid flow through the fluid outlet 108 to the direction show by arrow B.

In operation of the syringe 100 of figure 4, an external pressure is applied to the plunger 112 to insert the plunger head into the chamber 104 until the plunger head 116 contacts the end wall 110 of the hollow barrel 102. A vacuum is created in the region of the chamber 104 between the plunger head 116 and the closure means 118. A needle (not shown) is attached to the needle attachment means 106.

The needle tip is inserted into a body and the external pressure on the plunger 112 can be released. The vacuum in the region of the chamber 104 causes the plunger head 116 to be withdrawn slightly from the fully inserted condition. A partial vacuum is thus formed between the needle tip and the plunger head. The vacuum in the region of the chamber 104 will not cause the plunger head 116 to be fully withdrawn because the needle tip is in contact with solid or substantially solid material which cannot be sucked through the needle into the syringe 100.

When the needle tip contacts a liquid (not shown), liquid is drawn into the chamber 104, and the plunger 112 becomes fully withdrawn by means of the vacuum in the region of the chamber 104. The liquid then drains out of the syringe 100 via the fluid outlet 108 when external pressure is reapplied to the rod 114. Preferably, the rod 114 is pushed into the fully inserted condition.

The reader's attention is directed to all papers and documents which are filed concurrently with or previous to this specification in connection with this application and which are open to public inspection with this specification, and the contents of all such papers and documents are incorporated herein by reference.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings), may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment(s). The invention extend to any novel one, or any novel combination, of the features disclosed in this specification (including any accompanying claims, abstract and drawings), or to any novel one, or any novel combination, of the steps of any method or process so disclosed.

## Claims

1. A syringe comprising a hollow barrel, providing a chamber, having a needle attachment means and a plunger which is moveable within said chamber, said plunger comprising a plunger head and a rod, said plunger head being locatable inside said chamber, said plunger head being adapted to form a fluid tight fit with a wall of said chamber, and a closure means being arranged such that when said plunger is located within said chamber, said closure means is situated to the opposite side of said plunger head to which said needle attachment means is located, said closure means forming a fluid tight fit between said chamber and said plunger rod, wherein said needle attachment means is fixedly located relative to said hollow barrel.

2. A syringe according to Claim 1, wherein said closure means comprises a collar.

3. A syringe according to anyone of the present claims, further comprising a fluid outlet.

4. A syringe according to Claim 3, wherein said fluid outlet is located between said needle attachment means and the other end of said hollow body.

5. A syringe according to Claim 3 or 4, wherein said fluid outlet is located at end wall of said hollow barrel.

6. A syringe according to anyone of Claims 3-5, wherein said fluid outlet and/or said needle attachment means comprises valve means.

7. A syringe according to Claim 6, wherein, in use thereof, the direction of fluid flow through said needle attachment means is from the exterior of the needle into said chamber.

8. A syringe according to Claim 6 or 7, wherein in use thereof, the direction of fluid flow through said fluid outlet is from said chamber to the exterior of said hollow barrel.

9. A method of operating a syringe in accordance with any one of the preceding claims comprising the following steps:
(a) attaching a needle to said needle attachment means;
(b) locating said plunger head of said plunger inside the chamber;
(c) applying and maintaining an external pressure on said plunger in order to move said plunger head into contact with said end wall of said hollow barrel;
(d) inserting said needle into a body;
(e) releasing the external pressure on said plunger, and
(f) guiding said needle into said body until the tip of said needle contacts a liquid reservoir,

10. A method according to Claim 9, wherein if said syringe comprises a fluid outlet, said method further comprises the step of:
(g) reapplying an external pressure to the plunger in order to force liquid collected into said chamber out of said fluid outlet.
